(19) Europäisches Patentamt
European Patent Office
Office européen des brevets

(11) EP 3 615 508 B1

(12) **FASCICULE DE BREVET EUROPEEN**

(45) Date de publication et mention de la délivrance du brevet:
**09.12.2020 Bulletin 2020/50**

(21) Numéro de dépôt: **18725285.3**

(22) Date de dépôt: **24.04.2018**

(51) Int Cl.:
***C07C 69/612*** *(2006.01)* ***C09D 11/16*** *(2014.01)*

(86) Numéro de dépôt international:
**PCT/FR2018/051033**

(87) Numéro de publication internationale:
**WO 2018/197809 (01.11.2018 Gazette 2018/44)**

(54) **COMPOSITIONS DE PIGMENT THERMOCHROME**

THERMOCHROME PIGMENTZUSAMMENSETZUNGEN

THERMOCHROMIC PIGMENT COMPOSITIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.04.2017 FR 1753680**

(43) Date de publication de la demande:
**04.03.2020 Bulletin 2020/10**

(73) Titulaire: **Société BIC**
**92110 Clichy (FR)**

(72) Inventeurs:
- **DEBRAUWER, Christelle**
  **Quebec, G1S 3T3 (CA)**
- **DAMIANO, Anne-Lise**
  **13470 Carnoux-en-Provence (FR)**
- **BOURQUE, Alexander**
  **77144 Montevrain (FR)**
- **FOULONNEAU, François**
  **33800 Bordeaux (FR)**
- **CHOLLET, Guillaume**
  **33850 Leognan (FR)**

(74) Mandataire: **Cabinet Beau de Loménie**
**158, rue de l'Université**
**75340 Paris Cedex 07 (FR)**

(56) Documents cités:
**WO-A1-2016/198784**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 615 508 B1

Printed by Jouve, 75001 PARIS (FR)

## Description

La présente invention concerne des compositions de pigment thermochrome comprenant des composés spécifiques en tant que milieu réactionnel. La présente invention vise également des microcapsules de pigment thermochrome comprenant de telles compositions de pigment thermochrome, des compositions d'encre comprenant de telles microcapsules de pigment thermochrome, et enfin des instruments d'écriture comprenant de telles compositions d'encre.

## ETAT DE L'ART ANTÉRIEUR ET BUT DE L'INVENTION

Les compositions de pigment thermochrome présentent des propriétés de décoloration réversible liées à un changement de température. Ces compositions trouvent application lorsqu'un marquage à l'encre exige des effaçages répétés.

L'effet thermochrome d'une encre fonctionne grâce à l'association des trois composés suivants :

(A) au moins un composé organique colorant donneur d'électrons ou leuco-colorant,
(B) au moins un composé accepteur d'électrons ou développeur de couleur, et
(C) au moins un composé servant de milieu réactionnel capable de conduire à une réaction d'acceptation/don d'électrons réversible attribuable aux composés (A) et (B) ou agent régulateur de changement de température.

Les changements de température provoquent réversiblement la coloration ou la décoloration des encres. Ainsi, l'augmentation de chaleur va provoquer l'effacement de l'encre, tandis qu'un refroidissement provoquera son apparition. Ces changements suivent le schéma de la **Figure 1.** Sur ce schéma, la température de début de disparition de la couleur de l'encre est T3, celle à laquelle la couleur de l'encre a totalement disparue est T4 et TG est la température médium entre T3 et T4. A l'inverse, la température de début de réapparition de la couleur de l'encre est T2, celle à laquelle la couleur de l'encre a totalement réapparu est T1 et TH est la température se trouvant au milieu entre T1 et T2. On appelle largeur d'hystérésis de changement de couleur ($\Delta H$), la plage entre (TH) et (TG). WO2016/198784 divulgue des compositions de pigment thermochrome.

De manière surprenante, les Inventeurs ont découverts des composés spécifiques susceptibles d'être utilisés comme milieu réactionnel dans des compositions de pigment thermochrome, lesdites compositions permettant la préparation de microcapsules de pigment thermochrome présentant des plages de températures de fusion et de cristallisation optimales correspondant respectivement aux températures de décoloration et de recoloration de ces compositions. Les composés de l'invention présentent ainsi de nombreux avantages à être utilisés comme agent régulateur de changement de température dans des encres thermochromes : ils présentent des caractéristiques d'hystérésis remarquables et un contraste de couleurs extrêmement élevé entre l'état coloré et l'état décoloré.

## DESCRIPTION DE L'INVENTION

Selon un premier aspect, la présente invention a pour objet une composition de pigment thermochrome comprenant :

(A) au moins un composé organique colorant donneur d'électrons,
(B) au moins un composé accepteur d'électrons, et
(C) au moins un composé répondant à la formule (I) suivante :

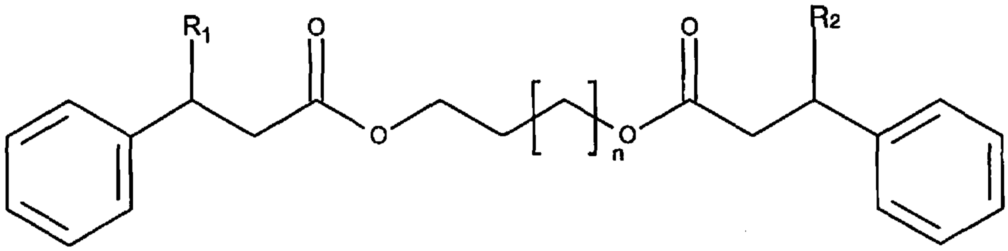

(I)

dans laquelle :

- $R_1$ représente H ou un groupe phényle,
- $R_2$ représente H ou un groupe phényle, et
- n = 1-8.

Dans la formule (I) ci-dessus, n peut être indépendamment choisi parmi un des entiers suivants : 1, 2, 3, 4, 5, 6, 7 ou 8.

Avantageusement, dans la formule (I) ci-dessus, $R_1$ et $R_2$ sont identiques.

Selon un premier mode de réalisation préféré, le composé (C) de l'invention répond à la formule ($I_a$) suivante :

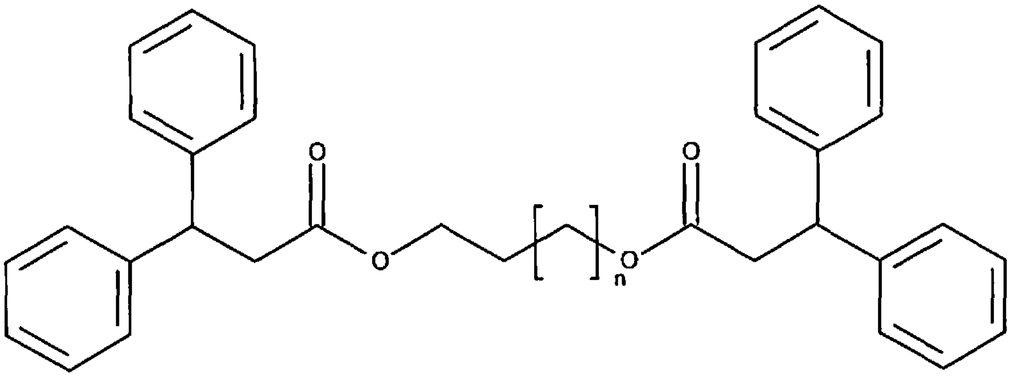

($I_a$)

dans laquelle n = 1-8.

Selon un deuxième mode de réalisation préféré, le composé (C) de l'invention répond à la formule ($I_b$) suivante :

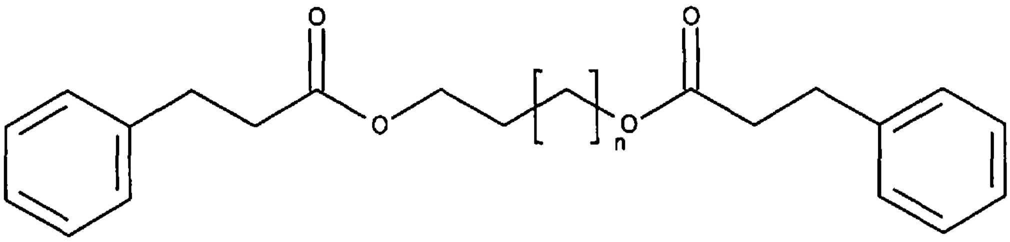

($I_b$)

dans laquelle n = 1-8.

Les composés de formule (I) de l'invention peuvent être synthétisés selon deux voies de synthèse différentes.

La première voie de synthèse répond au schéma réactionnel suivant (lorsque $R_1$ = $R_2$) :

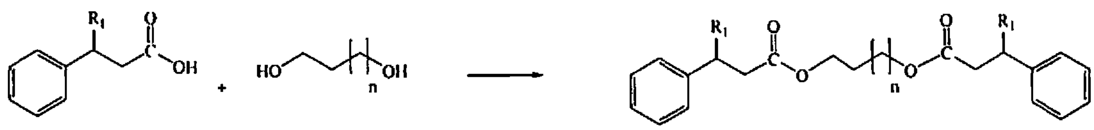

Dans cette première voie de synthèse, l'alcool possède un point de fusion inférieur à 60°C et joue le rôle de solvant. Il est utilisé en excès, de préférence à un ratio alcool/acide carboxylique de 1,5/1 à 3/1, et encore plus préférentiellement de 2/1. Le mélange d'alcool et d'acide carboxylique est chauffé en présence de catalyseur à une température allant de 120 à 200°C, et de préférence de 140 à 160°C, sous pression réduite, de préférence entre 200 et 800 mbar, jusqu'à ce que l'acide soit totalement consommé. Le catalyseur est de préférence un acide soluble dans l'eau, tel que l'acide paratoluènesulfonique. Le composé de formule (I) obtenu est ensuite purifié par recristallisation.

La deuxième voie de synthèse répond au schéma réactionnel suivant (lorsque $R_1$ = $R_2$) :

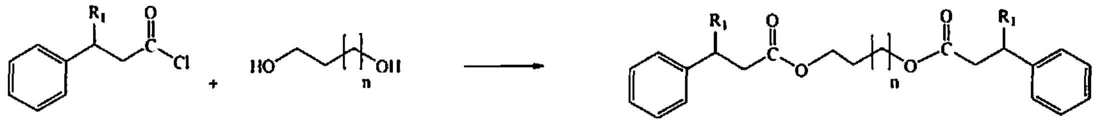

Dans cette deuxième voie de synthèse, l'alcool est solubilisé avec un catalyseur dans un solvant polaire aprotique, tel que le tétrahydrofurane (THF). Le catalyseur est de préférence une base volatile comme la triéthylamine. Le mélange est maintenu à froid, à une température allant de -20 à 20°C, préférentiellement de 0 à 10°C. Le mélange est maintenu à froid dans un bain de glace ou dans un bain de $CO_2$ solide plongé dans un solvant tel que l'acétone ou l'éthanol. Le mélange est avantageusement rendu inerte par ajout d'azote. Le chlorure d'acide est ensuite ajouté lentement, de préférence au goutte-à-goutte, pendant une durée allant de 15 à 60 minutes, de préférence en 30 minutes. Le ratio alcool/chlorure d'acide utilisé est de préférence de 1,1/1 à 1/1,1, et encore plus préférentiellement de 1/1. La température est ensuite élevée à température ambiante (25°C), et le mélange maintenu à cette température pendant 1 à 3 heures, de préférence pendant 2 heures, sous agitation. Le composé de formule (I) obtenu est ensuite purifié par recristallisation.

Le composé de formule (I) de l'invention est de préférence choisi parmi les composés suivants :

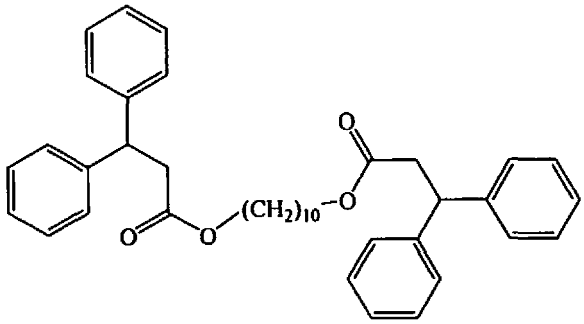

(1)

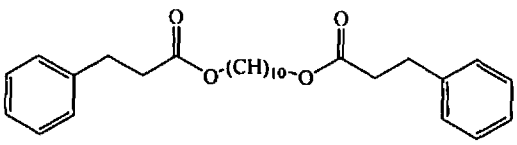

(2)

La température de fusion du composé de formule (I) de l'invention peut varier de 20 à 80°C, de préférence de 30 à 80°C, et encore plus préférentiellement de 40 à 70°C. C'est cette température de fusion optimale qui fait que le composé de formule (I) de l'invention est un composé idéal qui présente les propriétés requises pour être utilisé comme agent régulateur de changement de température dans des compositions de pigment thermochrome.

Dans la composition de pigment thermochrome de l'invention, les taux en poids des composés (A), (B) et (C) varient en fonction de la nature et de la concentration de chacun de ces composés.

Le taux en poids de composé organique colorant donneur d'électrons (A) peut varier de 1 à 10%, de préférence de 1 à 6%, et encore plus préférentiellement de 2 à 4%, en poids par rapport au poids total de la composition de pigment thermochrome.

Le taux en poids de composé accepteur d'électrons (B) peut varier de 1 à 20%, de préférence de 1 à 14%, et encore plus préférentiellement de 4 à 10%, en poids par rapport au poids total de la composition de pigment thermochrome.

Le taux en poids de composé (C) de formule (I) jouant le rôle de milieu réactionnel peut varier de 70 à 98%, de préférence de 80 à 98%, et encore plus préférentiellement de 86 à 94%, en poids par rapport au poids total de la composition de pigment thermochrome.

Ainsi, la composition de pigment thermochrome de l'invention peut comprendre :

(A) de 1 à 10%, de préférence de 1 à 6%, et encore plus préférentiellement de 2 à 4%, en poids d'au moins un composé organique colorant donneur d'électrons,

(B) de 1 à 20%, de préférence de 1 à 14%, et encore plus préférentiellement de 4 à 10%, en poids d'au moins un composé accepteur d'électrons, et

(C) de 70 à 98%, de préférence de 80 à 98%, et encore plus préférentiellement de 86 à 94%, en poids d'au moins

un composé répondant à la formule (I).

Selon un mode de réalisation préféré, la composition de pigment thermochrome de l'invention comprend :

(A) de 2 à 4% en poids d'au moins un composé organique colorant donneur d'électrons,
(B) de 4 à 10% en poids d'au moins un composé accepteur d'électrons, et
(C) de 86 à 94% en poids d'au moins un composé répondant à la formule (I).

Avantageusement, la composition de pigment thermochrome de l'invention présente une largeur d'hystérésis de changement de couleur (ΔH) après encapsulation allant de 20 à 80°C, de préférence de 30 à 80°C, et encore plus préférentiellement de 40 à 70°C.

En tant que composé organique colorant donneur d'électrons (A), des composés classiquement connus tels que les phtalides de diphénylméthane, les phtalides phénylindolyl, les indolylphthalides, les azaphthalides de diphénylméthane, les azaphthalides de phénylindolyl, les fluoranes, les styrylquinolines et les lactones diazarhodamine peuvent être cités, des exemples de ces composés étant présentés ci-après.

Le composé organique colorant donneur d'électrons (A) peut ainsi être choisi parmi le 3-(4-diéthylamino-2-éthoxyphényl)-3-(1-éthyl-2-méthylindol-3-yl)-4-azaphthalide (Blue 63, n° CAS : 69898-40-4), le 3,3-bis(p-diméthylaminophényl)-6-diméthylaminophthalate (n° CAS : 1552-42-7), le 2'-chloro-6'-(diéthylamino)-3'-méthylfluorane (n° CAS : 21121-62-0), le 6'-(diéthylamino)-1',3'-diméthylfluorane (n° CAS : 21934-68-9), le 2-chloro-6-(diéthylamino)-fluorane (n° CAS : 26567-23-7), le 3-diéthylaminobenzofluorane (n° CAS : 26628-47-7), le 3',6'-bis(diéthylamino)-2-(4-nitrophényl)spiro[isoindole-1,9'-xanthène]-3-one (n° CAS : 29199-09-5), le 2-phénylamino-3-méthyl-6-diéthylaminofluorane (n° CAS : 29512-49-0), le 2'-(dibenzylamino)-6'-(diéthylamino)fluorane (n° CAS : 34372-72-0), le 2-(2,4-diméthylphénylamino)-3-méthyl-6-diéthylaminofluorane (Black 15, n° CAS : 36431-22-8), le 3-(1,2-diméthyl-3-indolyl)-3-[4-(diéthylamino)-2-méthylphényl]phthalide (n° CAS : 36499-49-7), le 3',6'-diméthoxyfluorane (n° CAS : 36886-76-7), le 3,3-bis-(1-butyl-2-méthyl-indol-3-yl)-3H-isobenzofuran-1-one (Red 40, n° CAS : 50292-91-6), le 3,3-bis-(2-méthyl-1-octyl-1H-indol-3-yl)-3H-isobenzofuran-1-one (n° CAS : 50292-95-0), le 2'-anilino-6'-[éthyl(p-tolyl)amino]-3'-méthylspiro[isobenzofuran-1(3H),9'-[9H]xanthène]-3-one (n° CAS : 59129-79-2), le 3-(N-éthyl-n-isopentylamino)-6-méthyl-7-anilino fluorène (n° CAS : 70516-41-5), le 3-[4-(diéthylamino)phényl]-3-(1-éthyl-2-méthyl-1H-indol-3-yl)phthalide (n° CAS : 75805-17-3), le 2'-(2-chloroanilino)-6'-(dibutylamino)fluorane (n° CAS : 82137-81-3), le 2-phénylamino-3-méthyl-6-dibutylaminofluorane (n° CAS : 89331-94-2), le 3-(1-butyl-2-méthyl-1H-indol-3-yl)-6-(diméthylamino)-3-[4-(diméthylamino)phényl]-3-(1(3H)-isobenzofuranone (n° CAS : 92453-31-1), le 7-(4-diéthylamino-2-hexyloxyphényl)-7-(1-éthyl-2-méthyl-1H-indol-3-yl)-7H-furo[3,4-b]pyridin-5-one (Blue 203, n° CAS : 98660-18-5), le 7,7-bis[4-(diéthylamino)-2-éthoxyphényl]furo[3,4-b]pyridin-5-one (n° CAS : 132467-74-4), le N,N-diméthyl-4-[2-[2-(octyloxy)phényl]-6-phényl-4-pyridinyl]benzènamine (Yellow CK37, n° CAS : 144190-25-0), le 3-(2,2-bis(1-éthyl-2-méthylindol-3-yl)vinyl)-3-(4-diéthylaminophényl)-phthalide (n° CAS : 148716-90-9).

De manière préférée, le composé organique colorant donneur d'électrons (A) est choisi parmi le 3-(4-diéthylamino-2-éthoxyphényl)-3-(1-éthyl-2-méthylindol-3-yl)-4-azaphthalide (Blue 63, n° CAS : 69898-40-4), le 2'-(dibenzylamino)-6'-(diéthylamino)fluorane (n° CAS : 34372-72-0), le N,N-diméthyl-4-[2-[2-(octyloxy)phényl]-6-phényl-4-pyridinyl]benzènamine (Yellow CK37, n° CAS : 144190-25-0), le 7-(4-diéthylamino-2-hexyloxyphényl)-7-(1-éthyl-2-méthyl-1H-indol-3-yl)-7H-furo[3,4-b]pyridin-5-one (Blue 203, N° CAS : 98660-18-5), le 2-(2,4-diméthylphénylamino)-3-méthyl-6-diéthylaminofluoran (Black 15, N° CAS : 36431-22-8), et le 3,3-bis-(1-butyl-2-méthyl-indol-3-yl)-3H-isobenzofuran-1-one (Red 40, n° CAS : 50292-91-6).

En tant que composé accepteur d'électrons (B), on peut citer à titre non limitatif les composés ayant un proton actif tels que les composés ayant un groupe hydroxyle phénolique (monophénols ou polyphénols), leurs dérivés qui ont des substituants tels qu'un groupe alkyle, un groupe aryle, un groupe acyle, un groupe alcoxycarbonyle, un groupe carboxy, des esters de ceux-ci, un groupe amido ou un atome d'halogène, et les résines condensées phénol-aldéhyde tels que des bisphénols ou des trisphénols.

Au sens de la présente invention, on entend par :

- Alkyle : un groupe aliphatique hydrocarboné saturé, linéaire ou ramifié, en $C_1$-$C_{20}$, de préférence en $C_1$-$C_{12}$, plus préférentiellement en $C_1$-$C_6$, et encore plus préférentiellement en $C_1$-$C_4$. Le terme « ramifié » signifie qu'au moins un groupe alkyle inférieur tel qu'un méthyle ou un éthyle est porté par une chaîne alkyle linéaire. A titre de groupe alkyle, on peut mentionner par exemple les groupes méthyle, éthyle, n-propyle, i-propyle, n-butyle, t-butyle et n-pentyle.
- Aryle : tout groupe fonctionnel ou substituant dérivé d'au moins un cycle aromatique ; un cycle aromatique correspond à tout groupe mono- ou polycyclique plan comportant un système π délocalisé dans lequel chaque atome du cycle comporte une orbitale p, lesdites orbitales p se recouvrant les unes les autres ; parmi de tels groupes aryle, on peut mentionner les groupes phényle, biphényle, naphthalène et anthracène. Les groupes aryles de l'invention comprennent de préférence de 4 à 12 atomes de carbone, et de manière encore plus préférée de 5 à 6 atomes de carbone. De manière encore plus préférée, le groupe aryle de l'invention est un groupe phényle.

Ainsi, le composé accepteur d'électrons (B) peut être choisi parmi le 2,2-bis(4-hydroxy-3-méthylphényl)propane (Bisphénol C, n° CAS : 79-97-0), le 4-hexyl-1,3-dihydroxybenzène (4-hexylrésorcinol, n° CAS : 136-77-6), le 4,4'-cyclohexylidènebisphénol (BPZ, n° CAS : 843-55-0), le 4,4'-(hexafluoroisopropylidène)diphénol (Bisphénol AF, n° CAS : 1478-61-1), le 4,4'-(1-phényléthylidène)bisphénol (n° CAS : 1571-75-1), le 2,2'-dihydroxybiphényl (n° CAS : 1806-29-7), le 4,4'-éthylidènebisphénol (n° CAS : 2081-08-5), le 4,4'-(1,4-phénylènediisopropylidène)bisphénol (n° CAS : 2167-51-3), le 1,1-bis(4-hydroxy-3-méthylphényl)cyclohexane (n° CAS : 2362-14-3), le 9,9-bis(4-hydroxyphényl)fluorène (n° CAS : 3236-71-3), le 4,4'-(1,3-phénylènediisopropylidène)bisphénol (n° CAS : 13595-25-0), le 1,1,1-tris(4-hydroxyphényl)éthane (n° CAS : 27955-94-8), le 4,4'-(2-éthylhexylidène)diphénol (n° CAS : 74462-02-5), le α,α,α'-tris(4-hydroxyphényl)-1-éthyl-4-isopropylbenzène (n° CAS : 110726-28-8), le 4-(1,1,3,3-tétraméthylbutyl)phénol (n° CAS : 140-66-9), le 4-hydroxydiphényléther (n° CAS : 831-82-3), le bis(2-hydroxy-1-naphthyl)méthane (n° CAS : 1096-84-0), le 4-(méthylsulfonyl)phénol (n° CAS : 14763-60-1), le 4-hydroxyphényl-4'-isopropoxyphényl sulfone (n° CAS : 95235-30-6), le 4,4'-dihydroxybiphényl (n° CAS : 92-88-6), le 4-hydroxybiphényl (n° CAS : 92-69-3), le p-hydroxycumène (n° CAS : 99-89-8), le 2,4-dihydroxybenzophénone (n° CAS : 131-56-6), l'hydroquinone monométhyléther (MEHQ, n° CAS : 150-76-5), le 3-n-pentadécylphénol (n° CAS : 501-24-6), le 4-(2-phénylisopropyl)phénol (n° CAS : 599-64-4), le 5-chloro-2-(2,4-dichlorophénoxy)phénol (n° CAS : 3380-34-5), le N-(p-toluènesulfonyl)-N'-(3-(p-toluènesulfonyloxy)phényl)urée (n° CAS : 232938-43-1), le 2,2-bis(3,5-dibromo-4-hydroxyphényl)propane (n° CAS : 79-94-7), le 4,4'-isopropylidènediphénol (n° CAS : 80-05-7), et le 4,4'-sulfonyldiphénol, (BPS, n° CAS : 80-09-1).

De manière préférée, le composé accepteur d'électrons (B) est choisi parmi le 2,2-bis(4-hydroxy-3-méthylphényl)propane (Bisphénol C, n° CAS : 79-97-0), le 4-hexyl-1,3-dihydroxybenzène (4-hexylrésorcinol, n° CAS : 136-77-6), le 4,4'-cyclohexylidènebisphénol (BPZ, n° CAS : 843-55-0), le 4,4'-(hexafluoroisopropylidène)diphénol (Bisphénol AF, n° CAS : 1478-61-1), le 4,4'-(1-phényléthylidène)bisphénol (n° CAS : 1571-75-1), le 2,2'-dihydroxybiphényl (n° CAS : 1806-29-7), le 4,4'-(1,4-phénylènediisopropylidène)bisphénol (n° CAS : 2167-51-3), le 1,1-bis(4-hydroxy-3-méthylphényl)cyclohexane (n° CAS : 2362-14-3), le 9,9-bis(4-hydroxyphényl)fluorène (n° CAS : 3236-71-3), le 4,4'-(1,3-phénylènediisopropylidène)bisphénol (n° CAS : 13595-25-0), le 1,1,1-tris(4-hydroxyphényl)éthane (n° CAS : 27955-94-8), le 4,4'-(2-éthylhexylidène)diphénol (n° CAS : 74462-02-5), et le α,α,α'-tris(4-hydroxyphényl)-1-éthyl-4-isopropylbenzène (n° CAS : 110726-28-8).

La composition de pigment thermochrome de l'invention est préparée par dissolution des composés (A) et (B) dans le composé (C) de formule (I) de l'invention, puis agitation jusqu'à obtention d'un mélange homogène à l'aide d'un agitateur tel qu'un homo mélangeur ou un disperseur.

Les composés (A) et (B) ainsi associés avec le composé de formule (I) de l'invention peuvent être formulés sous la forme de microcapsules. Ainsi, la composition de pigment thermochrome de l'invention est encapsulée dans des microcapsules pour former des microcapsules de pigment thermochrome. De telles microcapsules de pigment thermochrome constituent un autre objet selon l'invention. Ils présentent des caractéristiques avantageuses dans la mesure où ils sont résistants aux contraintes mécaniques, insolubles et donc dispersables dans l'eau, et d'agglomération lente.

La température de fusion (ou température de décoloration T4) des microcapsules de pigment thermochrome de l'invention peut varier de 20 à 80°C, de préférence de 30 à 80°C, et encore plus préférentiellement de 40 à 70°C.

La température de cristallisation (ou température de recoloration T1) des microcapsules de pigment thermochrome de l'invention peut varier de -40 à 20°C, de préférence de -30 à 10°C, et encore plus préférentiellement de -20 à 0°C.

Les microcapsules de pigment thermochrome de l'invention présentent un diamètre moyen pouvant aller de 0,5 à 30 μm, de préférence de 1 à 10 μm, et encore plus préférentiellement de 3-5 μm. Ce diamètre moyen correspond au d90 en volume et signifie que 90% en volume des microcapsules sont constitués de microcapsules ayant une taille comprise dans l'intervalle indiquée. Ce diamètre moyen peut être déterminé par granulométrie laser en utilisant un appareil Zetasizer Nano ZS de Malvern Instruments.

Les procédés de micro-encapsulation utilisés incluent à titre non limitatif des méthodes conventionnelles, telles que :

- des procédés chimiques qui reposent sur la formation *in situ* des microcapsules enrobantes, comme par exemple par polymérisation ou polycondensation interfaciale, ces procédés étant les préférés,
- des procédés physico-chimiques, comme par exemple par séparation de phases ou coacervation, par évaporation-extraction de solvant, par gélification thermique d'émulsions (hot melt), ou
- des procédés mécaniques, comme par exemple par nébulisation/séchage (spray drying), par gélification ou congélation de gouttes, par enrobage en lit fluidisé (spray-coating).

Les microcapsules de pigment thermochrome de l'invention sont avantageusement à base de résine aminoplaste, et de préférence à base de résine mélamine, de résine urée ou de résine benzoguanamine.

Les microcapsules de pigment thermochrome de l'invention sont de préférence préparées par polymérisation *in situ* à partir de résine mélamine.

Un autre objet de l'invention vise une composition d'encre comprenant des microcapsules de pigment thermochrome selon l'invention.

Les microcapsules de pigment thermochrome de l'invention présentent au sein de la composition d'encre représentent de 5 à 50% en poids du poids total de la composition d'encre.

La composition d'encre de l'invention est par ailleurs majoritairement constituée d'eau. Avantageusement, l'eau représente 40 à 80% en poids du poids total de la composition d'encre.

La composition d'encre de l'invention peut également contenir un ou plusieurs co-solvants miscibles dans l'eau. Ainsi, la composition d'encre de l'invention peut contenir un solvant organique ou aqueux, de préférence un solvant aqueux.

Parmi les solvants qui peuvent être ajoutés à la composition d'encre de l'invention, on peut citer l'eau et les solvants polaires miscibles dans l'eau, comme par exemple :

- les alcools : les alcools linéaires ou ramifiés en $C_1$-$C_{15}$ tels que l'isopropanol, le butanol, l'isobutanol, le pentanol, l'alcool benzylique ; la glycérine ; la diglycérine ; la polyglycérine
- les esters tels que l'acétate d'éthyle ou l'acétate de propyle,
- les esters de carbonate tels que le carbonate de propylène ou le carbonate d'éthylène,
- les cétones telles que la méthylisobutylcétone (MIBC), l'acétone ou la cyclohexanone,
- les glycols tels que l'éthylène glycol, le diéthylène glycol, le triéthylène glycol, le propylène glycol, le polyéthylène glycol, l'éthylène glycol monométhyl éther, le 3-butylène glycol et le thiodiéthylène glycol,
- les amides tels que le diméthylacétamide ou le diméthylformamide, et
- leur mélange.

Le ou les solvants représentent de 5 à 20% en poids du poids total de la composition d'encre.

La composition d'encre de l'invention peut également contenir un ou plusieurs adjuvants spécifiques qui peuvent jouer différents rôles selon l'application finale visée. Ces applications peuvent concerner l'impression d'encre par sérigraphie, l'impression offset, l'impression par héliogravure, le revêtement par pulvérisation, le revêtement électrostatique, le revêtement par dépôt électrolytique, le revêtement au rouleau, l'impression au jet d'encre, les encres pour outils d'écriture tels que les stylos à bille, les stylos pinceaux, les marqueurs, les crayons de couleurs. La composition d'encre de l'invention peut également être ajoutée à une composition de résine thermoplastique ou thermodurcissable pour former des pièces moulées.

Parmi les adjuvants mentionnés ci-dessus, on peut citer :

- des modificateurs de rhéologie (agent rhéofluidifiant) capables de générer un effet gélifiant, tels que la gomme de xanthane ou la gomme arabique,
- des antimousses, tels que les dispersions aqueuses de polysiloxane modifiés (MOUSSEX® de Synthron),
- des régulateurs de pH, tels que l'hydroxyde de sodium, la triéthanolamine,
- des tensioactifs, tels que les polyéthers polyols (TERGITOL™ de DOW),
- des biocides, tels que les isothiazolinones (ACTICIDE® de Thor),
- des anticorrosifs, tel que la benzotriazole,
- des lubrifiants,
- des dispersants,
- des agents de coalescence,
- des agents réticulants,
- des agents mouillants,
- des plastifiants,
- des antioxydants,
- des stabilisateurs UV.

Un objet supplémentaire de l'invention concerne des instruments d'écriture comprenant une composition d'encre selon l'invention. Ces instruments sont généralement constitués d'un corps comprenant la composition d'encre de l'invention, et éventuellement un élément de frottement. L'instrument d'écriture selon l'invention est avantageusement choisi parmi les stylos à bille, les crayons, les craies, et encore plus avantageusement les stylos à bille à encre effaçable par friction. L'élément de frottement de l'instrument d'écriture est de préférence une gomme.

Les supports sur lesquels peut être appliquée la composition d'encre de l'invention sont le papier, les fibres,

le cuir, le plastique, le verre, le métal, le bois, le béton.

Outre les dispositions qui précèdent, l'invention comprend encore d'autres dispositions qui ressortiront du complément de description qui suit, qui se rapporte à la synthèse de composés de formule (I) selon l'invention, à leur caractérisation, et à leur utilisation comme agent régulateur de changement de température dans des compositions de pigment thermochrome.

**EXEMPLES** :

Les composés (1) et (2) de formules suivantes :

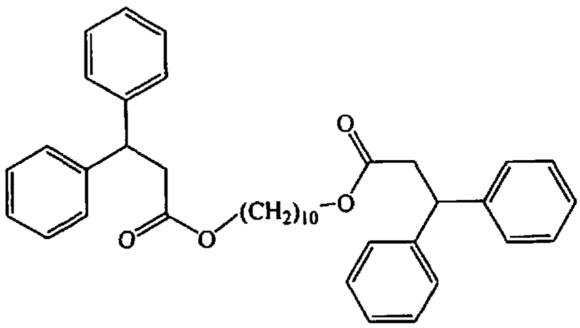

(1)

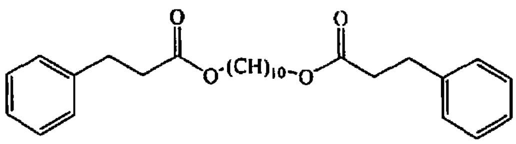

(2)

sont préparés selon les protocoles suivants :

Synthèse du composé (1) :

10 g de 1,10-décanediol (n° CAS 112-47-0), 52 g d'acide 3,3-diphénylpropionique (n° CAS 606-83-7), et 200 mg d'acide p-toluènesulfonique monohydraté (APTS) (n° CAS 6192-52-5) sont mélangés et chauffés à 140°C pendant 6 heures, sous pression réduite (400 mbar).

Le milieu réactionnel est ensuite solubilisé dans 150 mL d'acétate d'éthyle. La phase organique est récupérée et lavée avec trois fois 150 mL d'eau. La phase organique est ensuite séchée sur du sulfate de sodium, et le solvant évaporé.

Le produit est recristallisé une fois par de l'isopropanol, et une fois par de l'éthanol. L'analyse par chromatographie en phase gazeuse (GPC) du produit obtenu révèle que le produit est pur à 95%.

Synthèse du composé (2) :

10 g de 1,10-décanediol (n° CAS 112-47-0), 34,2 g d'acide 3-phénylpropionique (n° CAS 501-52-0), et 200 mg d'acide p-toluènesulfonique monohydraté (APTS) (n° CAS 6192-52-5) sont mélangés et chauffés à 160°C pendant 6 heures, sous pression réduite (400 mbar).

Le milieu réactionnel est ensuite solubilisé dans 150 mL d'acétate d'éthyle. La phase organique est récupérée et lavée avec trois fois 150 mL d'eau. La phase organique est ensuite séchée sur du sulfate de sodium, et le solvant évaporé.

Le produit est recristallisé une fois par de l'isopropanol, et une fois par de l'éthanol. L'analyse par chromatographie en phase gazeuse (GPC) du produit obtenu révèle que le produit est pur à 95%.

Les températures de fusion $T_{FUS}$ des composés (1) et (2) obtenus sont mesurées par calorimétrie différentielle à balayage (DSC) grâce à un appareil TA Instruments Q20, sur une plage de température allant de -50 à 100°C, à des vitesses de chauffage/refroidissement de +/-20°C/minute. Les températures mesurées sont indiquées dans le **Tableau 1** ci-après.

**Tableau 1** :

| Composé de formule (I) | $T_{FUS}$ (°C) |
|---|---|
| Composé (1) | 71 |
| Composé (2) | 35 |

Préparation d'une composition de pigment thermochrome :

Une composition de pigment thermochrome est préparée en mélangeant 1,9 parts en poids de 3-(4-diéthylamino-2-éthoxyphényl)-3-(1-éthyl-2-méthylindol-3-yl)-4-azaphthalide (composé (A), n° CAS : 69898-40-4), 2,1 parts en poids de 4,4'-(hexafluoroisopropylidène)diphénol (composé (B1) n° CAS : 1478-61-1), 2,1 parts en poids de 2,2-bis(4-hydroxy-3-méthylphenyl)propane (composé (B2), n° CAS : 79-97-0), et 93,9 parts en poids d'un composé de formule (I) selon l'invention (composé (1) (composé (C)) :

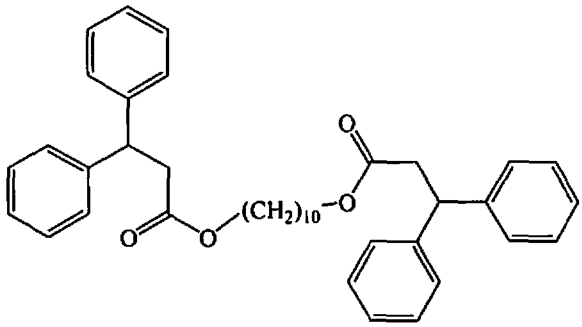

(1)

Le mélange obtenu est chauffé, sous agitation, à une température de 110°C pendant 45 minutes, jusqu'à solubilisation complète des composés (A), (B1) et (B2) dans le composé (C).

Préparation des microcapsules de pigment thermochrome :

7,7 parts en poids d'une solution aqueuse d'un copolymère d'anhydride maléique et de méthylvinyléther (solution à 33% en poids de copolymère) sont neutralisés avec 10,5 parts en poids d'une solution aqueuse d'hydroxyde de sodium (solution à 1,0 M). Cette solution est diluée avec 39,5 parts en poids d'eau, et le mélange émulsionné avec un homogénéiseur à une vitesse d'au moins 15 $m.s^{-1}$. 25,3 parts en poids de la composition de pigment thermochrome précédemment préparée sont ajoutés, et l'émulsion obtenue est maintenue à une température de 90°C pendant 30 minutes. 17,0 parts en poids d'un pré-polymère mélamine-formaldéhyde (solution aqueuse à 50% en poids de pré-polymère) sont ensuite ajoutés au mélange au goutte-à-goutte. Le milieu réactionnel est alors chauffé à une température de 90°C et mélangé à une vitesse d'au moins 15 $m.s^{-1}$ pendant 4 heures.

Un slurry constitué de microcapsules de pigment thermochrome dispersées dans un solvant aqueux est obtenu, les microcapsules présentant un diamètre d90 de 3,8 μm, déterminé à l'aide d'un système Zetasizer Nano ZS de Malvern avec une illumination à 632 nm.

Les microcapsules de pigment thermochrome obtenues ont la propriété de changer de couleur du bleu à l'incolore au-dessus de 71°C avec un effet d'hystérésis de la couleur.

**Détermination des températures de décoloration et de recoloration des microcapsules de pigment thermochrome préparées :**

Les températures de transition des microcapsules de pigment thermochrome obtenues sont mesurées par calorimétrie différentielle à balayage (DSC) grâce à un appareil TA Instruments Q20, sur une plage de température allant de -50 à 100°C, à des vitesses de chauffage/refroidissement de +/-20°C/minute. Les températures mesurées sont indiquées dans le **Tableau 2** ci-après.

**Tableau 2** : Températures de transition des microcapsules de pigment thermochrome préparées

| | Changement de couleur coloré ↔ incolore | T1 (°C) | T2 (°C) | T3 (°C) | T4 (°C) | $T_H$ (°C) | $T_G$ (°C) | ΔH |
|---|---|---|---|---|---|---|---|---|
| Microcapsules de pigment thermochrome préparées | bleue ↔ incolore | -24 | -10 | 40 | 71 | -17 | 55 | 72 |

Les températures de transition mesurées sont les suivantes :

T1 : température de recoloration complète,
T2 : température de recoloration partielle,
T3 : température de décoloration partielle,
T4 : température de décoloration complète, $T_H = \frac{T1+T2}{2}$, $T_G = \frac{T3+T4}{2}$,
ΔH = plage d'hystérésis = $T_G - T_H$.

## Revendications

**1.** Composition de pigment thermochrome comprenant :

(A) au moins un composé organique colorant donneur d'électrons,
(B) au moins un composé accepteur d'électrons, et
(C) au moins un composé répondant à la formule (I) :

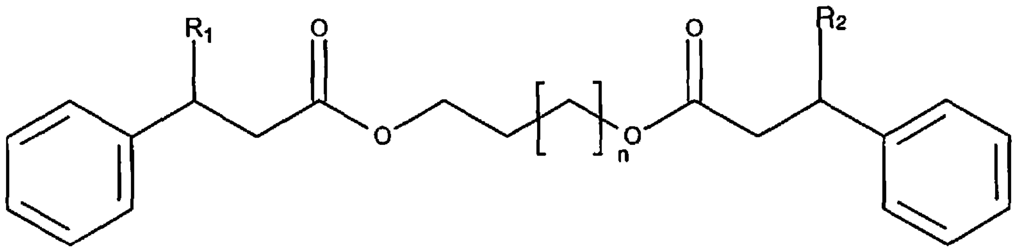

(I)

dans laquelle :

- $R_1$ représente H ou un groupe phényle,
- $R_2$ représente H ou un groupe phényle, et
- n = 1-8.

**2.** Composition de pigment thermochrome selon la revendication 1, dans laquelle le composé (C) répond à la formule ($I_a$) suivante :

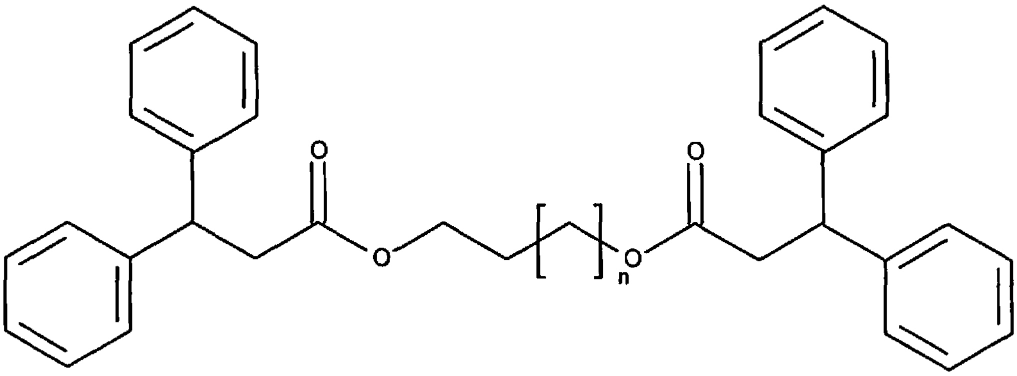

$(I_a)$

dans laquelle n = 1-8.

3. Composition de pigment thermochrome selon la revendication 1, dans laquelle le composé (C) répond à la formule ($I_b$) suivante :

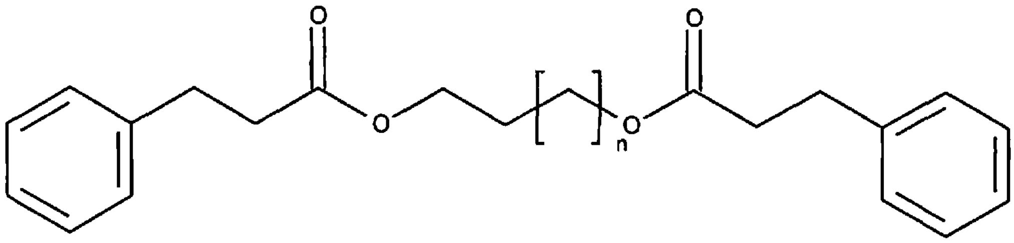

$(I_b)$

dans laquelle n = 1-8.

4. Composition selon l'une des revendications 1 à 3, dans laquelle le composé (A) est choisi parmi le 3-(4-diéthylamino-2-éthoxyphényl)-3-(1-éthyl-2-méthylindol-3-yl)-4-azaphthalide (Blue 63, n° CAS : 69898-40-4), le 2'-(dibenzylamino)-6'-(diéthylamino)fluorane (n° CAS : 34372-72-0), le N,N-diméthyl-4-[2-[2-(octyloxy)phényl]-6-phényl-4-pyridinyl]benzènamine (Yellow CK37, n° CAS : 144190-25-0), le 7-(4-diéthylamino-2-hexyloxyphényl)-7-(1-éthyl-2-méthyl-1H-indol-3-yl)-7H-furo[3,4-b]pyridin-5-one (Blue 203, N° CAS : 98660-18-5), le 2-(2,4-diméthylphénylamino)-3-méthyl-6-diéthylaminofluoran (Black 15, N° CAS : 36431-22-8), et le 3,3-bis-(1-butyl-2-méthyl-indol-3-yl)-3H-isobenzofuran-1-one (Red 40, n° CAS : 50292-91-6).

5. Composition selon l'une des revendications 1 à 4, dans laquelle le composé (B) est choisi parmi le 2,2-bis(4-hydroxy-3-méthylphényl)propane (Bisphénol C, n° CAS : 79-97-0), le 4-hexyl-1,3-dihydroxybenzène (4-hexylrésorcinol, n° CAS : 136-77-6), le 4,4'-cyclohexylidènebisphénol (BPZ, n° CAS : 843-55-0), le 4,4'-(hexafluoroisopropylidène)diphénol (Bisphénol AF, n° CAS : 1478-61-1), le 4,4'-(1-phényléthylidène)bisphénol (n° CAS: 1571-75-1), le 2,2'-dihydroxybiphényl (n° CAS : 1806-29-7), le 4,4'-(1,4-phénylènediisopropylidène)bisphénol (n° CAS : 2167-51-3), le 1,1-bis(4-hydroxy-3-méthylphényl)cyclohexane (n° CAS : 2362-14-3), le 9,9-bis(4-hydroxyphényl)fluorène (n° CAS : 3236-71-3), le 4,4'-(1,3-phénylènediisopropylidène)bisphénol (n° CAS : 13595-25-0), le 1,1,1-tris(4-hydroxyphényl)éthane (n° CAS : 27955-94-8), le 4,4'-(2-éthylhexylidène)diphénol (n° CAS : 74462-02-5), le a,a,a'-tris(4-hydroxyphényl)-1-éthyl-4-isopropylbenzène (n° CAS : 110726-28-8).

6. Microcapsule de pigment thermochrome comprenant une composition selon l'une des revendications 1 à 5.

7. Composition d'encre comprenant des microcapsules de pigment thermochrome selon la revendication 6.

8. Instrument d'écriture comprenant une composition d'encre selon la revendication 7.

9. Instrument d'écriture selon la revendication 8 choisi parmi les stylos à bille à encre effaçable par friction.

**Patentansprüche**

1. Thermochrome Pigmentzusammensetzung, umfassend:

(A) mindestens eine organische Farbstoffverbindung, die ein Elektronendonor ist,
(B) mindestens eine Elektronenakzeptorverbindung und
(C) mindestens eine Verbindung, die der Formel (I) entspricht:

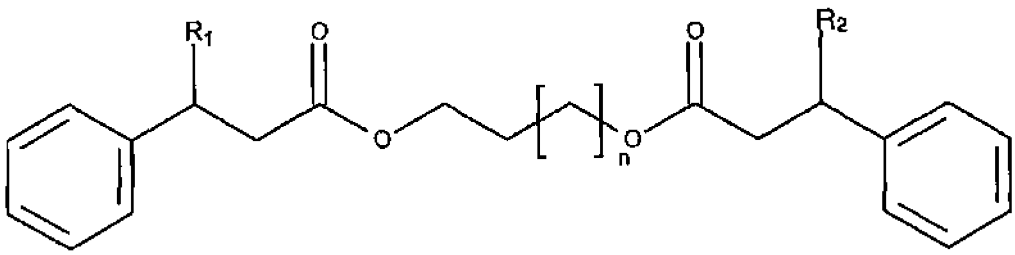

(I)

wobei:

- $R_1$ H oder eine Phenylgruppe darstellt,
- $R_2$ H oder eine Phenylgruppe darstellt und
- n = 1-8.

2. Thermochrome Pigmentzusammensetzung nach Anspruch 1, wobei die Verbindung (C) der folgenden Formel ($I_a$) entspricht:

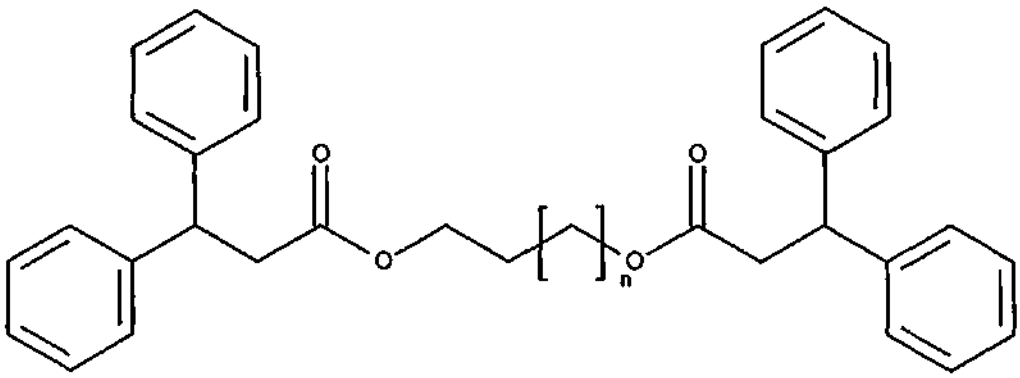

($I_a$)

wobei n = 1-8.

3. Thermochrome Pigmentzusammensetzung nach Anspruch 1, wobei die Verbindung (C) der folgenden Formel ($I_b$) entspricht:

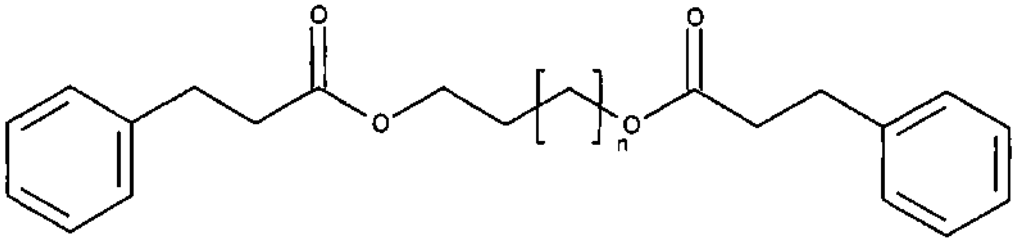

(I_b)

wobei n = 1-8.

4. Zusammensetzung nach einem der Ansprüche 1 bis 3, wobei die Verbindung (A) ausgewählt ist aus 3-(4-Diethylamino-2-ethoxyphenyl)-3-(1-ethyl-2-methylindol-3-yl)-4-azaphthalid (Blau 63, CAS-Nr.: 69898-40-4), 2-(Dibenzylamino)-6'-(diethylamino)fluoran (CAS-Nr.: 34372-72-0), N,N-Dimethyl-4-[2-[2-(octyloxy)phenyl]-6-phenyl-4-pyridinyl]benzolamin (Gelb CK37, CAS-Nr.: 144190-25-0), 7-(4-Diethylamino-2-hexyloxyphenyl)-7-(1-ethyl-2-methyl-1H-indol-3-yl)-7H-furo[3,4-b]pyridin-5-on (Blau 203, CAS-Nr.: 98660-18-5), 2-(2,4-Dimethylphenylamino)-3-methyl-6-diethylaminofluoran (Schwarz 15, CAS-Nr.: 36431-22-8) und 3,3-Bis-(1-butyl-2-methylindol-3-yl)-3H-isobenzofuran-1-on (Rot 40, CAS-Nr.: 50292-91-6).

5. Zusammensetzung nach einem der Ansprüche 1 bis 4, wobei die Verbindung (B) ausgewählt ist aus 2,2-Bis(4-hydroxy-3-methylphenyl)propan (Bisphenol C, CAS-Nummer: 79-97-0), 4-Hexyl-1,3-dihydroxybenzol (4-Hexylresorcin, CAS-Nr.: 136-77-6), 4,4'-Cyclohexylidenbisphenol (BPZ, CAS-Nr.: 843-55-0), 4,4'-(Hexafluorisopropyliden)diphenol (Bisphenol AF, CAS-Nr.: 1478-61-1), 4,4'-(1-Phenylethyliden)bisphenol (CAS-Nr.: 1571-75-1), 2,2'-Dihydroxybiphenyl (CAS-Nummer: 1806-29-7), 4,4'-(1,4-Phenylendiisopropyliden)bisphenol (CAS-Nr.: 2167-51-3), 1,1-Bis(4-hydroxy-3-methylphenyl)cyclohexan (CAS-Nr.: 2362-14-3), 9,9-Bis(4-hydroxyphenyl)fluoren (CAS-Nummer: 3236-71-3), 4,4'-(1,3-Phenylendiisopropyliden)bisphenol (CAS-Nummer: 13595-25-0), 1,1,1-Tris(4-hydroxyphenyl)ethan (CAS-Nr.: 27955-94-8), 4,4'-(2-Ethylhexyliden)diphenol (CAS-Nr.: 74462-02-5), α,α,α'-Tris(4-hydroxyphenyl)-1-ethyl-4-isopropylbenzol (CAS-Nr.: 110726-28-8).

6. Mikrokapsel des thermochromen Pigments, umfassend eine Zusammensetzung nach einem der Ansprüche 1 bis 5.

7. Tintenzusammensetzung, umfassend Mikrokapseln des thermochromen Pigments nach Anspruch 6.

8. Schreibgerät, umfassend eine Tintenzusammensetzung nach Anspruch 7.

9. Schreibgerät nach Anspruch 8, ausgewählt aus reibungslöschbaren Tintenkugelschreibern .

## Claims

1. Thermochromic pigment composition, comprising:

   (A) at least one organic electron donor dye compound,
   (B) at least one electron acceptor compound, and
   (C) at least one compound corresponding to formula (I):

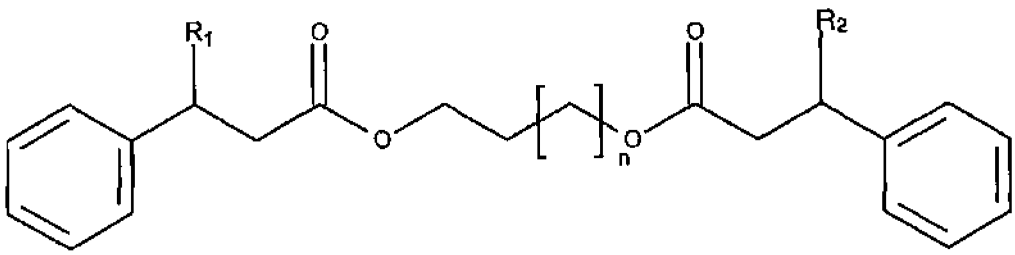

(I)

in which:

- $R_1$ represents H or a phenyl group,
- $R_2$ represents H or a phenyl group, and
- n = 1-8.

2. Thermochromic pigment composition according to claim 1, wherein the compound (C) corresponds to the following formula ($I_a$):

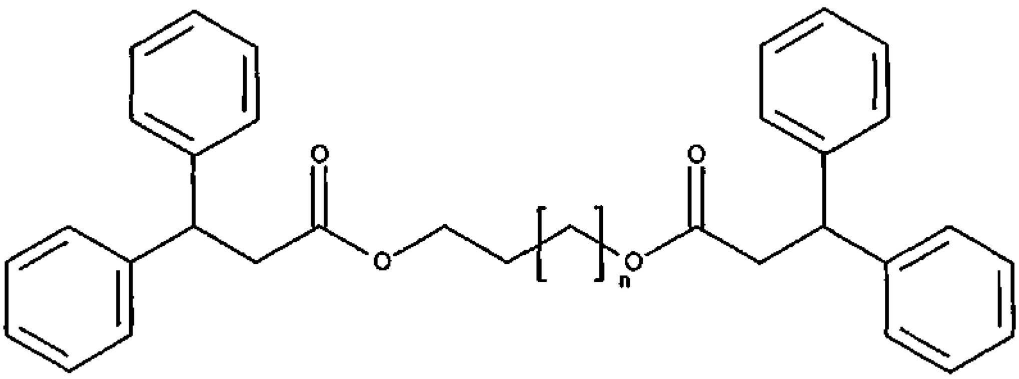

($I_a$)

in which n = 1-8.

3. Thermochromic pigment composition according to claim 1, wherein the compound (C) corresponds to the following formula ($I_b$):

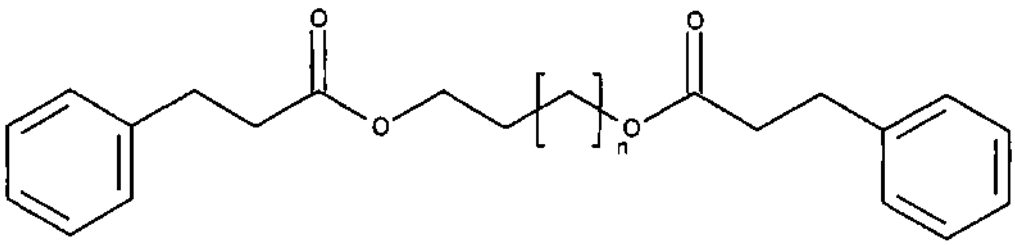

($I_b$)

in which n = 1-8.

4. Composition according to any of claims 1 to 3, wherein the compound (A) is selected from 3-(4-diethylamino-2-ethoxyphenyl)-3-(1-ethyl-2-methylindol-3-yl)-4-azaphthalide (Blue 63, CAS number: 69898-40-4), 2-(dibenzylamino)-6'-(diethylamino)fluoran (CAS number: 34372-72-0), N,N-dimethyl-4-[2-[2-(octyloxy)phenyl]-6-phenyl-4-pyridinyl]benzenamine (Yellow CK37, CAS number: 144190-25-0), 7-(4-diethylamino-2-hexyloxyphenyl)-7-(1-ethyl-2-methyl-1H-indol-3-yl)-7H-furo[3,4-b]pyridin-5-one (Blue 203, CAS number: 98660-18-5), 2-(2,4-dimethylphenylamino)-3-methyl-6-diethylaminofluoran (Black 15, CAS number: 36431-22-8), and 3,3-bis-(1-butyl-2-methyl-indol-3-yl)-3H-isobenzofuran-1-one (Red 40, CAS number: 50292-91-6).

**5.** Composition according to any of claims 1 to 4, wherein the compound (B) is selected from 2,2-bis(4-hydroxy-3-methylphenyl)propane (Bisphenol C, CAS number: 79-97-0), 4-hexyl-1,3-dihydroxybenzene (4-hexylresorcinol, CAS number: 136-77-6), 4,4'-cyclohexylidenebisphenol (BPZ, CAS number: 843-55-0), 4,4'-(hexafluoroisopropylidene)diphenol (Bisphenol AF, CAS number: 1478-61-1), 4,4'-(1-phenylethylidene)bisphenol (CAS number: 1571-75-1), 2,2'-dihydroxybiphenyl (CAS number: 1806-29-7), 4,4'-(1,4-phenylenediisopropylidene)bisphenol (CAS number: 2167-51-3), 1,1-bis(4-hydroxy-3-methylphenyl)cyclohexane (CAS number: 2362-14-3), 9,9-bis(4-hydroxyphenyl)fluorene (CAS number: 3236-71-3), 4,4'-(1,3-phenylenediisopropylidene)bisphenol (CAS number: 13595-25-0), 1,1,1-tris(4-hydroxyphenyl)ethane (CAS number: 27955-94-8), 4,4'-(2-ethylhexylidene)diphenol (CAS number: 74462-02-5), $\alpha,\alpha,\alpha'$-tris(4-hydroxyphenyl)-1-ethyl-4-isopropylbenzene (CAS number: 110726-28-8).

**6.** Thermochromic pigment microcapsule, comprising a composition according to any of claims 1 to 5.

**7.** Ink composition, comprising thermochromic pigment microcapsules according to claim 6.

**8.** Writing instrument, comprising an ink composition according to claim 7.

**9.** Writing instrument according to claim 8, selected from friction-erasable ink ballpoint pens.

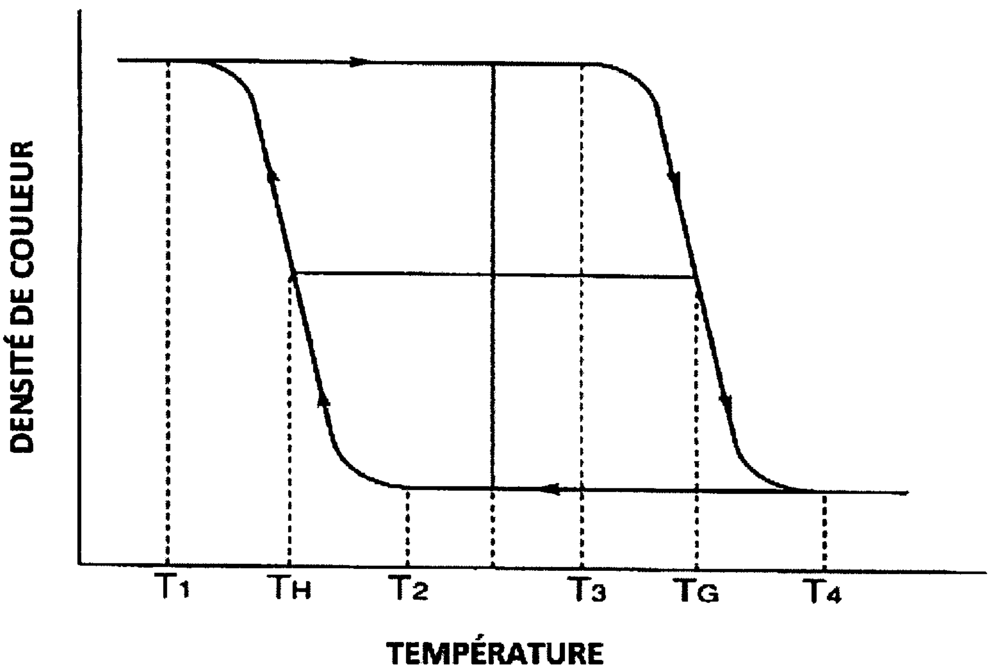


FIGURE 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2016198784 A


### Littérature non-brevet citée dans la description


- *CHEMICAL ABSTRACTS,* 69898-40-4
- *CHEMICAL ABSTRACTS,* 1552-42-7
- *CHEMICAL ABSTRACTS,* 21121-62-0
- *CHEMICAL ABSTRACTS,* 21934-68-9
- *CHEMICAL ABSTRACTS,* 26567-23-7
- *CHEMICAL ABSTRACTS,* 26628-47-7
- *CHEMICAL ABSTRACTS,* 29199-09-5
- *CHEMICAL ABSTRACTS,* 29512-49-0
- *CHEMICAL ABSTRACTS,* 34372-72-0
- *CHEMICAL ABSTRACTS,* 36431-22-8
- *CHEMICAL ABSTRACTS,* 36499-49-7
- *CHEMICAL ABSTRACTS,* 36886-76-7
- *CHEMICAL ABSTRACTS,* 50292-91-6
- *CHEMICAL ABSTRACTS,* 50292-95-0
- *CHEMICAL ABSTRACTS,* 59129-79-2
- *CHEMICAL ABSTRACTS,* 70516-41-5
- *CHEMICAL ABSTRACTS,* 75805-17-3
- *CHEMICAL ABSTRACTS,* 82137-81-3
- *CHEMICAL ABSTRACTS,* 89331-94-2
- *CHEMICAL ABSTRACTS,* 92453-31-1
- *CHEMICAL ABSTRACTS,* 98660-18-5
- *CHEMICAL ABSTRACTS,* 132467-74-4
- *CHEMICAL ABSTRACTS,* 144190-25-0
- *CHEMICAL ABSTRACTS,* 148716-90-9
- *CHEMICAL ABSTRACTS,* 79-97-0
- *CHEMICAL ABSTRACTS,* 136-77-6
- *CHEMICAL ABSTRACTS,* 843-55-0
- *CHEMICAL ABSTRACTS,* 1478-61-1
- *CHEMICAL ABSTRACTS,* 1571-75-1
- *CHEMICAL ABSTRACTS,* 1806-29-7
- *CHEMICAL ABSTRACTS,* 2081-08-5
- *CHEMICAL ABSTRACTS,* 2167-51-3
- *CHEMICAL ABSTRACTS,* 2362-14-3
- *CHEMICAL ABSTRACTS,* 3236-71-3
- *CHEMICAL ABSTRACTS,* 13595-25-0
- *CHEMICAL ABSTRACTS,* 27955-94-8
- *CHEMICAL ABSTRACTS,* 74462-02-5
- *CHEMICAL ABSTRACTS,* 110726-28-8
- *CHEMICAL ABSTRACTS,* 140-66-9
- *CHEMICAL ABSTRACTS,* 831-82-3
- *CHEMICAL ABSTRACTS,* 1096-84-0
- *CHEMICAL ABSTRACTS,* 14763-60-1
- *CHEMICAL ABSTRACTS,* 95235-30-6
- *CHEMICAL ABSTRACTS,* 92-88-6
- *CHEMICAL ABSTRACTS,* 92-69-3
- *CHEMICAL ABSTRACTS,* 99-89-8
- *CHEMICAL ABSTRACTS,* 131-56-6
- *CHEMICAL ABSTRACTS,* 150-76-5
- *CHEMICAL ABSTRACTS,* 501-24-6
- *CHEMICAL ABSTRACTS,* 599-64-4
- *CHEMICAL ABSTRACTS,* 3380-34-5
- *CHEMICAL ABSTRACTS,* 232938-43-1
- *CHEMICAL ABSTRACTS,* 79-94-7
- *CHEMICAL ABSTRACTS,* 80-05-7
- *CHEMICAL ABSTRACTS,* 80-09-1
- *CHEMICAL ABSTRACTS,* 112-47-0
- *CHEMICAL ABSTRACTS,* 606-83-7
- *CHEMICAL ABSTRACTS,* 6192-52-5
- *CHEMICAL ABSTRACTS,* 501-52-0